# EUROPEAN PATENT APPLICATION

(11) **EP 4 350 701 A1**
(43) Date of publication of application: **10.04.2024**
(21) Application number: 22200219.8
(22) Date of filing: 07.10.2022
(51) Int. Cl.: G16H 10/00, G16H 50/20

(54) **CLASSIFYING INDIVIDUALS SUFFERING FROM CHRONIC COUGH**

(71) Applicant: SIVA Health AG, 8003 Zürich (CH)
(72) Inventor: JANBAKHSHI, Parvaneh, 51373 Leverkusen (DE); MOHAMMADI, Seyed, Sadegh, 51373 Leverkusen (DE); RECHSTEINER, Daniel, 51373 Leverkusen (DE)
(74) Representative: Maiwald GmbH

(57) **Abstract**

Systems, methods, and computer programs disclosed herein relate to training and using a machine learning model for classifying individuals suffering from chronic cough at least partially based on information about cough events of the patient.

## Description

### TECHNICAL FIELD

Systems, methods, and computer programs disclosed herein relate to training and using a machine learning model for classifying individuals suffering from chronic cough at least partially based on information about cough events of the patient.

### BACKGROUND

Chronic cough is defined in adults as a cough that lasts for more than 8 weeks. When it proves intractable to standard-of-care treatment, it can be referred to as refractory chronic cough (RCC).

Over the past decade, significant scientific progress has been made in understanding the mechanisms of chronic cough, leading to several clinical drug development programs investigating potential novel antitussive agents. These efforts are most advanced in the area of purinergic receptors, of which the P2X class function as ligand-gated ion channels that are responsive to ATP. In the airways, purines, including ATP, can trigger reflexes by activating vagal sensory nerves, and ATP can sensitize airway sensory nerves to tussive stimuli. The P2X3 homotrimer and the P2X2/3 heterotrimer have been implicated as most relevant in cough induction.

The P2X3 receptor antagonist gefapixant showed a significant cough-suppressing effect in phase 2 studies, but at the expense of a reduction in taste sensation in a significant subset of subjects.

The P2X3 receptor antagonist eliapixant achieved a statistically significant reduction in the 24-hour cough count (average hourly cough frequency based on 24-hour sound recordings) of up to 27% over placebo after 12 weeks of treatment. However, eliapixant is not being pursued because the overall benefit does not outweigh the risks.

There is still a need to find treatments (e.g., drugs) for chronic cough.

For the development of new drugs and for the use of drugs in therapy, it is important to determine whether individual patients respond to the (new) drug.

There can be many reasons why an individual patient may not respond to a medication. In order to avoid side effects and not to burden the individual patient unnecessarily, it should be detected as early as possible whether the patient responds to the drug or not.

### SUMMARY

This is achieved by the subject matter of the independent claims of the present disclosure. Preferred embodiments are defined in the dependent claims, the description and the drawings.

In a first aspect, the present disclosure relates to a computer-implemented method of classifying an individual suffering from chronic cough, the method comprising:
- providing a machine learning model, wherein the machine learning model is configured and trained on training data to assign a patient to one of two classes, a first class representing patients who received medications for chronic cough, and a second class representing patients who received placebos, the training data comprising, for each patient of a multitude of patients, information about cough events of the patient before and after starting treatment with the medication or after receiving the placebos, respectively,
- receiving patient data, wherein the patient data comprise information about cough events of the individual before and after initiating therapy for chronic cough,
- inputting the patient data into the trained machine learning model,
- receiving information about which class the individual has been assigned to from the machine learning model,
- outputting the information.

In another aspect, the present disclosure provides a computer system comprising:
a processor; and
a memory storing an application program configured to perform, when executed by the processor, an operation, the operation comprising:
   - providing a machine learning model, wherein the machine learning model is configured and trained on training data to assign a patient to one of two classes, a first class representing patients who received medications for chronic cough, and a second class representing patients who received placebos, the training data comprising, for each patient of a multitude of patients, information about cough events of the patient before and after starting treatment with the medication or after receiving the placebos, respectively,
   - receiving patient data, wherein the patient data comprise information about cough events of the individual before and after initiating therapy for chronic cough,
   - inputting the patient data into the trained machine learning model,
   - receiving information about which class the individual has been assigned to from the machine learning model,
   - outputting the information.

In another aspect, the present disclosure provides a non-transitory computer readable storage medium having stored thereon software instructions that, when executed by a processor of a computer system, cause the computer system to execute the following steps:
- providing a machine learning model, wherein the machine learning model is configured and trained on training data to assign a patient to one of two classes, a first class representing patients who received medications for chronic cough, and a second class representing patients who received placebos, the training data comprising, for each patient of a multitude of patients, information about cough events of the patient before and after starting treatment with the medication or after receiving the placebos, respectively,
- receiving patient data, wherein the patient data comprise information about cough events of the individual before and after initiating therapy for chronic cough,
- inputting the patient data into the trained machine learning model,
- receiving information about which class the individual has been assigned to from the machine learning model,
- outputting the information.

### DETAILED DESCRIPTION

The invention will be more particularly elucidated below without distinguishing between the aspects of the invention (method, computer system, computer-readable storage medium). On the contrary, the following elucidations are intended to apply analogously to all the aspects of the invention, irrespective of in which context (method, computer system, computer-readable storage medium) they occur.

If steps are stated in an order in the present description or in the claims, this does not necessarily mean that the invention is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

In a study it was found that patients who suffered from refractory chronic cough and were treated with the P2X3 receptor antagonist eliapixant could be distinguished from patients who also suffered from refractory chronic cough but received placebos, based on measurable patient data. While a majority of patients having received placebos showed no improvement in cough over time, a majority patients treated with eliapixant showed a change in cough behavior.

If patients treated with eliapixant can be distinguished from patients who received placebos based on measurable data, patients who respond to cough therapy must also be distinguishable from patients who do not respond to cough therapy based on analogous data.

Thus, the present invention is based on a machine learning model that has been trained using training data from patients who have received a drug and patients who have received a placebo. However, assuming efficacy of medication for patients in the training data, the trained machine learning model can be used not only to detect whether new patients have received the drug or placebo, but also to detect whether new patients are responding to a certain cough therapy.

Therefore, the present invention provides means for determining for an individual whether the individual is responding to a therapy for cough.

The present invention likewise provides means for determining an improvement in an individual's cough.

The present invention likewise provides means for determining for an individual whether the individual received a drug for cough or an ineffective drug.

The present invention also provides means for an individual to determine whether he or she has received a sufficient dose of a drug for cough or whether the dose was too low.

The present invention makes use of a machine learning model. Such a "machine learning model", as used herein, may be understood as a computer implemented data processing architecture. The machine learning model can receive input data and provide output data based on that input data and on parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

The machine learning model of the present disclosure is or comprises a machine learning classifier (or classifier for short). A "classifier" in machine learning is an algorithm that automatically orders or categorizes data into one or more of a set of "classes". In the case of the present invention, a model is trained to discriminate patients who have received medication for chronic cough from patients who have received placebo. So, in case of the present invention, there are at least two classes, a first class representing patients suffering from chronic cough who have received medication for chronic cough, and a second class representing patient suffering from chronic cough who have received placebo.

The machine learning classifier of the present disclosure may be any classifier that is suitable for computer-based machine learning. A non-limiting example includes a random forest machine learning classifier.

Random forest is an ensemble learning method for classification, regression and other tasks that operates by constructing a multitude of decision trees at training time. For classification tasks, the output of the random forest is the class selected by most trees.

Numerous options are available for performing random forest training and generating a prediction from a random forest classifier. As a non-limiting example, R programming language may be used. Other classifiers may also be used in accordance with aspects of the present disclosure as well, including, without limitation, an artificial neural network classifier, a support vector machine, a max entropy classifier, an extreme gradient boosting classifier, and a random fern classifier.

The classifier is trained to assign a patient to one of the at least two classes at least partially based on information about cough events of the patient before and after starting treatment with a medication or after receiving placebos, respectively.

The classifier is trained on training data. The training data comprise, for each patient of a multitude of patients, patient data (as input data) and an information whether the respective patient received medication for chronic cough or placebos (as target data).

The term "multitude of patients" means at least ten patients, preferably more than one hundred. Some of the patients (e.g., 20% or 30% or 40% or any other percentage) received placebos, the other patients received medication.

The patient data comprise information about cough events of each patient during a first time period before and during one or more second periods after starting treatment with a medication or after taking placebos, respectively.

During treatment, the patient is usually administered the drug in the form of a fixed dose once or several times a day, or the patient applies it himself.

Patients receiving placebos are usually given them or self-administer them at the same times as patients receiving the medication (drug).

For example, the first time period may be 48 hours or 36 hours or 24 hours or 12 hours or 8 hours. For example, each second time period may be 8 hours or 12 hours or 24 hours or 36 hours or 48 hours or 3 days or 4 days or 5 days.

There may be one or more second time periods. The second time periods may extend over a period of days or weeks. Further on in the description, an example is given of how a number of four 'second time periods' can be distributed over a period of 84 days after the start of treatment (or after the start of receiving placebos). However, the invention is not limited to this example.

Information about cough events may be or comprise one or more of the following:
- total number of coughs in one or more periods of time (e.g., in the first and/or in the one or more second periods of time),
- number of coughs in a period of defined length before a first dose of medication or placebo,
- number of coughs in one or more periods of defined length after starting treatment with medication or placebo, respectively,
- decrease in total number of coughs after starting treatment with medication or placebo, compared with the number of coughs before having received a first dose of medication or placebo,
- total number of coughs while awake (e.g., before and/or after starting treatment with medication or placebo, respectively),
- decrease in total number of coughs while awake (before vs. after starting treatment with medication or placebo, respectively),
- intervals between cough events (e.g., before and/or after starting treatment with medication or placebo, respectively).

Instead of and/or in addition to absolute numbers, mean values, relative values, standard deviations and/or other statistical variables can be used, such as:
- the average number of coughs in a defined period of time (e.g., per hour or per day) before and/or after starting treatment with medication or placebo,
- the relative decrease in the number of coughs (before versus after starting treatment with medication or placebo),
- the average number of coughs in a defined period of time while awake (e.g., before and/or after starting treatment with medication or placebo, respectively),
- the relative decrease in the number of coughs while awake (before versus after starting treatment with medication or placebo, respectively),
- average interval between cough events (e.g., before and/or after starting treatment with medication or placebo, respectively).

Such cough information can be obtained, e.g., from audio recordings.

Audio recordings can be generated by means of an audio recorder.

For example, the audio recorder may be an audio recorder worn by a patient in or on the body that continuously records ambient sounds to detect cough events. Such cough monitoring devices are described in the prior art (see, e.g., WO2021/099614, WO2021/154105, US7727161).

Methods for automatic cough detection and cough counting based on audio recordings are also described in the prior art (see, e.g., WO2008/131387A2, WO2009/153681A1, WO2021/234037A1).

However, cough events can be detected not only on the basis of audio recordings; it is also conceivable that the cough events are detected on the basis of a signal other than an audio signal or on the basis of different (more than one) signals.

In general, a "signal" is a function that conveys information about a phenomenon and/or an event. Preferably, a signal is a change in a physical quantity over time. Signal data is a digital representation of said signal.

Coughing produces sound that can be detected as an acoustic signal (audio signal). At the same time, coughing leads to movements of at least the upper body, which can be detected, e.g., as a visible signal by a camera and/or as an electrical signal by means of acceleration sensors. In addition, cough produces electromyographic signals.

The way in which a signal is generated, present and/or detected is also referred to as the "modality" of the signal. To stay with the example of coughing, a cough event usually generates an acoustic signal (audio signal), an acceleration signal and an electrocardiogram signal and other metrologically detectable signals that can be used for detection, identification and/or characterization of the event causing the signals. The acoustic signal, the acceleration signal and the electromyographic signal are examples of signals of different modalities.

For example, US20190336039 discloses the detection of cough events by means of a piezoelectric sensor; EP4044916 discloses the detection of cough events based on an electrogram signal and an accelerometer signal.

Thus, the present invention is not limited to a certain modality that detects cough events.

The patient data can also comprise further patient data such as age, gender, body size, body weight, body mass index, ethnics, resting heart rate, heart rate variability, blood pressure, sugar concentration in urine, body temperature, impedance (e.g., thoracic impedance), lifestyle information about the life of the patient, such as consumption of alcohol, smoking, and/or exercise and/or the patient's diet, medical intervention parameters such as regular medication, occasional medication, or other previous or current medical interventions and/or other information about the patient's previous and/or current treatments and/or reported health conditions and/or combinations thereof.

Patient data may comprise information about a person's condition obtained from the person himself/herself (self-assessment data, (electronic) patient reported outcome data (e)PRO)).

Patient data may comprise one or more medical images. A medical image is a visual representation of the human body or a part thereof.

Techniques for generating medical images include X-ray radiography, computerized tomography, fluoroscopy, magnetic resonance imaging, ultrasonography, endoscopy, elastography, tactile imaging, thermography, microscopy, positron emission tomography and others.

Examples of medical images include CT (computer tomography) scans, X-ray images, MRI (magnetic resonance imaging) scans, fluorescein angiography images, OCT (optical coherence tomography) scans, histopathological images, ultrasound images and others.

Instead of and/or in addition to the absolute or relative frequencies of cough events, audio recordings themselves or other signal recordings themselves or features extracted from the audio recordings and/or signal recordings of individual cough events can also be fed to the classifier.

For example, the classifier may be part of an artificial neural network trained to extract features from digital audio recordings or other signal data or medical images or other patient data and then perform classification based on the extracted features (e.g., in the form of a feature vector).

As used herein, "feature extraction" is a process of dimensionality reduction by which an initial set of data is reduced to more manageable groups for processing. Feature extraction starts from the initial set of data and builds derived values (features) intended to be informative and non-redundant. A characteristic of these initial data sets is a large number of variables that require a lot of computing resources to process. Feature extraction is the name for methods that select and/or combine variables into features, effectively reducing the amount of data that must be processed, while still accurately and completely describing the initial data set.

The artificial neural network can have different input layers for different modalities of input data. The data from each modality can be fed to a feature extraction unit. Each feature extraction unit can be configured and trained to generate a feature vector. A "feature vector" is a *k*-dimensional vector of numerical features that represent an object or a subject (in the present case a patient and/or one or more cough events), wherein k is an integer greater than 0. The term "feature vector" shall also include single values, matrices, tensors, and the like.

The different feature vectors can be combined into one feature vector, for example, by concatenating or by a pooling operation. Examples of pooling operations are max pooling and average pooling. Max pooling uses the maximum value of each dimension of the feature vectors, while average pooling takes the average value. Details on the topic of pooling can be found in textbooks on artificial neural networks, see, e.g., S. Khan et al.: A Guide to Convolutional Neural Networks for Computer Vision, Morgan & Claypool Publishers 2018, ISBN 9781681730226, in particular chapter 4.2.3.

Attention mechanisms can be used to give more weight to individual features than to other features. In machine learning, attention is a technique that mimics cognitive attention. The effect enhances some parts of the input data while diminishing other parts - the thought being that the machine learning model should devote more focus to that small but important part of the data. Learning which part of the data is more important than others is trained in the training phase.

Attention mechanism are described in scientific articles and patents / patent applications (see e.g.: arXiv:1807.03380v1 [cs.CV] 9 Jul 2018, arXiv: 1911.07559v2 [cs.CV] 5 Dec 2019, arXiv:1904.02874v3 [cs.LG] 12 Jul 2021, arXiv:2006.03347v1 [cs.CV] 5 Jun 2020, DOI: 10.3390/fi11010009, EP3166049A1, US20170262996, WO2020/222985).

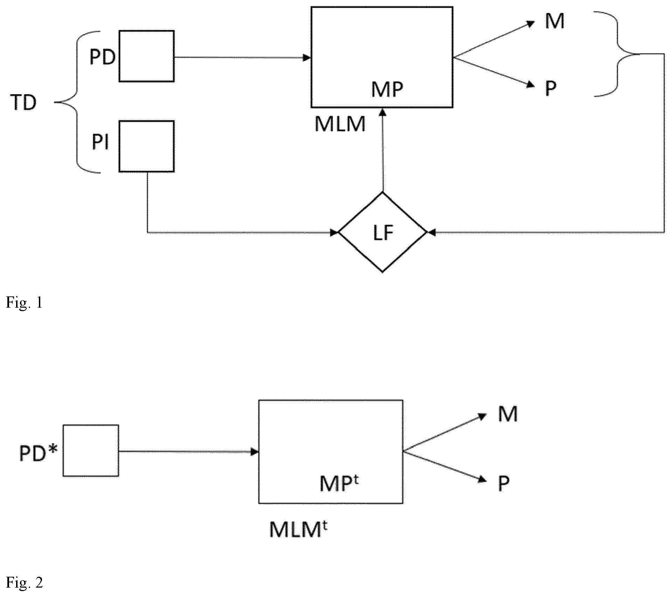

Fig. 1 shows schematically by way of example, the process of training the machine learning model of the present disclosure.

The machine learning model MLM is trained on training data TD. The training data TD comprise, for each patient of a multitude of patients, patient data PD, and information PI about whether the patient received medication or placebos. The patient data PD comprise information about cough events of the patient before and after starting treatment with medication or a placebo, respectively.

The patient data PD are used as input data of the machine learning model. The information PI about whether the patient received medication or placebo is used as target data. The patient data PD are inputted into the machine learning model MLM. The machine learning model MLM is configured to assign the patient data PD to one of two classes, a first class M representing patients who received medication and a second class P representing patients who received placebos.

The class output of the machine learning model MLM is compared with the target data PI. A loss function LF can be used to quantify the deviation. Model parameters MP of the machine learning model MLM can be modified in an optimization procedure (such as a gradient descent procedure) to reduce the deviation.

Fig. 2 shows schematically by way of example, how the trained machine learning model of the present disclosure can be used to classify individuals suffering from chronic cough.

Patient data PD^{∗} of an individual are inputted into the trained machine learning model MLM^{t}. The trained machine learning model MLM^{t} may have been trained as described with reference to Fig. 1. The patient data PD^{∗} comprise information about cough events of the individual before and after starting a therapy for chronic cough.

For example, a therapy for chronic cough may include the taking of one or more medications, a surgical procedure, treatment with electromagnetic radiation, with radioactive radiation, with electric current, and/or any other measure.

The trained machine learning model MLM^{t} is trained and configured to assign the individual to one of two classes, a first class M representing patients who received a medication and a second class representing patients who received a placebo. The classification is done based on the patient data PD^{∗}.

If the individual is assigned to the first class M, it may mean that the individual shows improvement in cough. If the individual is assigned to the second class P, it may mean that the individual does not show improvement in cough. The classification result is outputted, e.g., displayed on a monitor and/or outputted via a loudspeaker and/or printed out with a printer and/or stored in a data memory.

If the individual has been assigned to the first class M, the classification result may mean that the medical intervention has been successful. It is conceivable that the corresponding therapy will be continued.

If the individual has been assigned to the second class P, the classification result may mean that the medical intervention has been unsuccessful. For example, the individual may not respond to an administered medication as desired. It may be that an administered dose has been too low. A physician who is shown the classification result may take another/additional action to alleviate the chronic cough.

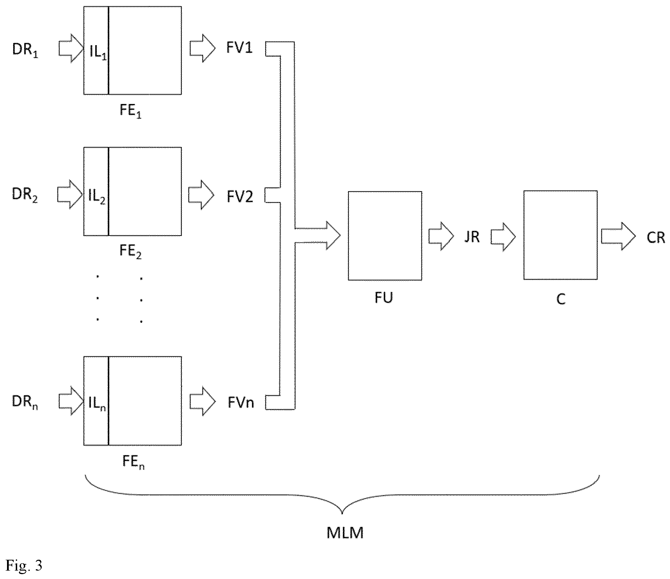

Fig. 3 shows schematically by way of example an embodiment of a machine learning model of the present disclosure.

The machine learning model MLM comprises a number n of feature extraction units FE₁, FE₂, ..., FEₙ. Each feature extraction unit provides an input layer IL for inputting patient data of different modalities, such as audio recordings, cough frequencies, medical images, electrogram data, accelerometer data, and/or other/further data. The input layers are indicated by the reference signs IL₁, IL₂, ..., ILₙ. The input data are indicated by the reference signs DR₁, DR₂, ..., DRₙ. Each feature extraction unit is configured to extract features from the input data and generate a feature vector. The feature vectors are indicated by the reference signs FV1, FV2, ..., FVn. The feature vectors are inputted into a feature vector combination unit FU. The feature vector combination unit FU is configured to generate a joint representation JR from the feature vectors. The joint representation JR is inputted into a classifier C. The classifier C is configured to generate a classification result CR.

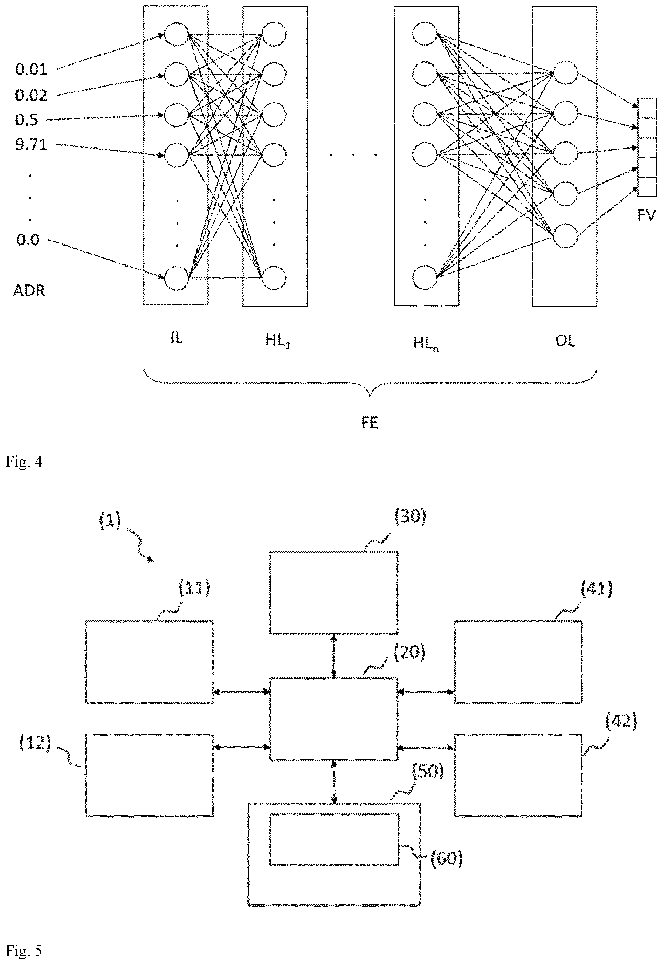

Fig. 4 shows schematically by way of example a feature extraction unit in the form of an artificial neural network. The feature extraction unit FE comprises an input layer IL, a number n of hidden layers HL₁ to HLₙ and an output layer. The input neurons of the input layer IL serve to receive patient data, e.g., a representation ADR of audio data. The output neurons serve to output a feature vector FV.

The neurons of the input layer IL and the hidden layer HL₁ are connected by connection lines having a connection weight, and the neurons of the hidden layer HLₙ and the output layer are also connected by connection lines with a connection weight. Similarly, the neurons of the hidden layers are connected to the neurons of neighboring hidden layers in a predetermined manner (not shown in Fig. 4). The connection weights can be learned through training.

The operations in accordance with the teachings herein may be performed by at least one computer system specially constructed for the desired purposes or at least one general-purpose computer system specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

A "computer system" is a system for electronic data processing that processes data by means of programmable calculation rules. Such a system usually comprises a "computer", that unit which comprises a processor for carrying out logical operations, and also peripherals.

In computer technology, "peripherals" refer to all devices which are connected to the computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

Computer systems of today are frequently divided into desktop PCs, portable PCs, laptops, notebooks, netbooks and tablet PCs and so-called handhelds (e.g., smartphone); all these systems can be utilized for carrying out the invention.

The term "process" as used above is intended to include any type of computation or manipulation or transformation of data represented as physical, e.g., electronic, phenomena which may occur or reside, e.g., within registers and/or memories of at least one computer or processor. The term processor includes a single processing unit or a plurality of distributed or remote such units.

Fig. 5 illustrates a computer system (1) according to some example implementations of the present disclosure in more detail.

Generally, a computer system of exemplary implementations of the present disclosure may be referred to as a computer and may comprise, include, or be embodied in one or more fixed or portable electronic devices. The computer may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs, which may be stored onboard the processing unit (20) or otherwise stored in the memory (50) of the same or another computer.

The processing unit (20) may be a number of processors, a multi-core processor or some other type of processor, depending on the particular implementation. For example, it may be a central processing unit (CPU), a field programmable gate array (FPGA), a graphics processing unit (GPU) and/or a tensor processing unit (TPU). Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program to perform one or more functions, the processing unit (20) of various examples may be capable of performing one or more functions without the aid of a computer program. In either instance, the processing unit (20) may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory (50) may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

The machine learning model, the trained machine learning model and the training data may be stored in the memory (50).

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions (60) may be stored in memory (50), and executed by processing unit (20) that is thereby programmed, to implement functions of the systems, subsystems, tools and their respective elements described herein. As will be appreciated, any suitable program code instructions (60) may be loaded onto a computer or other programmable apparatus from a computer-readable storage medium to produce a particular machine, such that the particular machine becomes a means for implementing the functions specified herein. These program code instructions (60) may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions (60) may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions (60) may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions (60) may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code instructions (60) stored in the memory (50). It will also be understood that one or more functions, and combinations of functions, may be implemented by special purpose hardware-based computer systems and/or processing circuitry which perform the specified functions, or combinations of special purpose hardware and program code instructions.

### Example

In a clinical study (L. McGarvey et al.: Efficacy and safety of eliapixant in refractory chronic cough: Results of the PAGANINI 12-week, randomized, placebo-controlled Phase 2b study, European Respiratory Journal Sep 2021, 58 (suppl 65) PA562), the cough events of 262 patients were counted. The cough events were counted 24 hours before the intake of a first medication or placebo (baseline), 24 hours after a first treatment monitoring visit, 24 hours after a second treatment monitoring visit, 24 hours after a third treatment monitoring visit, and 24 hours after a fourth treatment monitoring visit.

The first treatment monitoring visit took place on average 14 days after the start of the treatment.

The second treatment monitoring visit took place on average 28 days after the start of the treatment.

The third treatment monitoring visit took place on average 57 days after the start of the treatment.

The fourth treatment monitoring visit took place on average 84 days after the start of the treatment.

Patients received either eliapixant as medication or placebos.

191 patients received eliapixant (25 mg to 150 mg) twice daily as medication; the remaining 71 patients received placebos.

The following cough information were used as input data for training a machine learning model:
- total number of coughs
- relative decrease in total number of cough events with respect to the baseline (for each 24 h period after each treatment monitoring visit)
- area under curve (AUC) of normalized total number of coughs
- average hourly awake cough, i.e., during awake time
- relative decrease in average hourly awake cough with respect to baseline (for each 24 h period after each treatment monitoring visit)
- AUC of normalized average hourly awake cough
- mean and standard deviations of cough time interval series

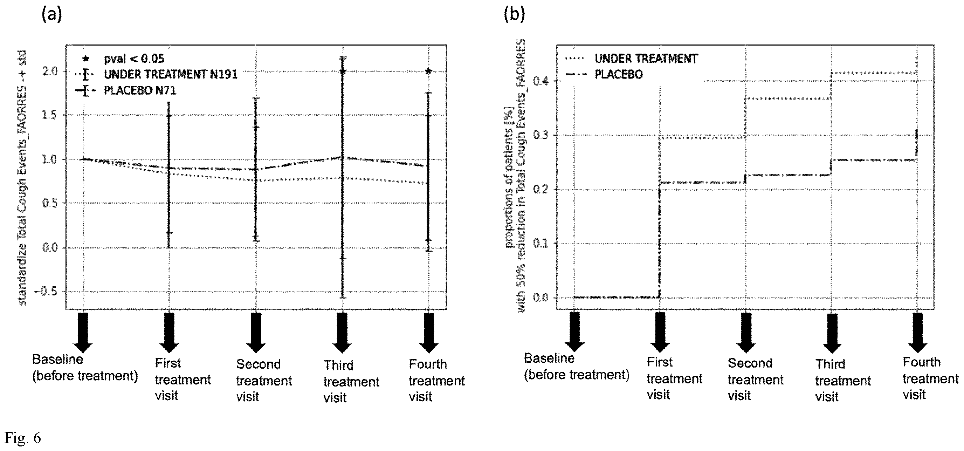

Fig. 6 (a) shows the average and standard deviation of the normalized total number of cough events for patients having received medication and patients having received placebos as a function of treatment monitoring visit.

Fig. 6 (b) shows the proportion of patients with 50% reduction in total number of cough events. as a function of treatment monitoring visit.

The machine learning model was a random forest (RF) classifier.

An RF classifier was considered where the minimum number of samples required to split an internal node is 2. For other hyperparameters to be tuned, we considered a range of [5,10,15,20,50,100] for the number of trees in the forest and a range of [2,5,7,9] for the maximum depth of the tree. Nested cross-validation (4 foldings) was performed on the training data to tune the hyperparameters.

We considered a RF classifier with the minimum number of samples required to split an internal node being 2, and for other hyper-parameters to be tuned a range of [5,10,15,20,50,100] for the number of trees in the forest and a range of [2,5,7,9] for the maximum depth of the tree was considered. For hyper-parameters tuning a grid search nested cross validation (4 folds) on train data was used.

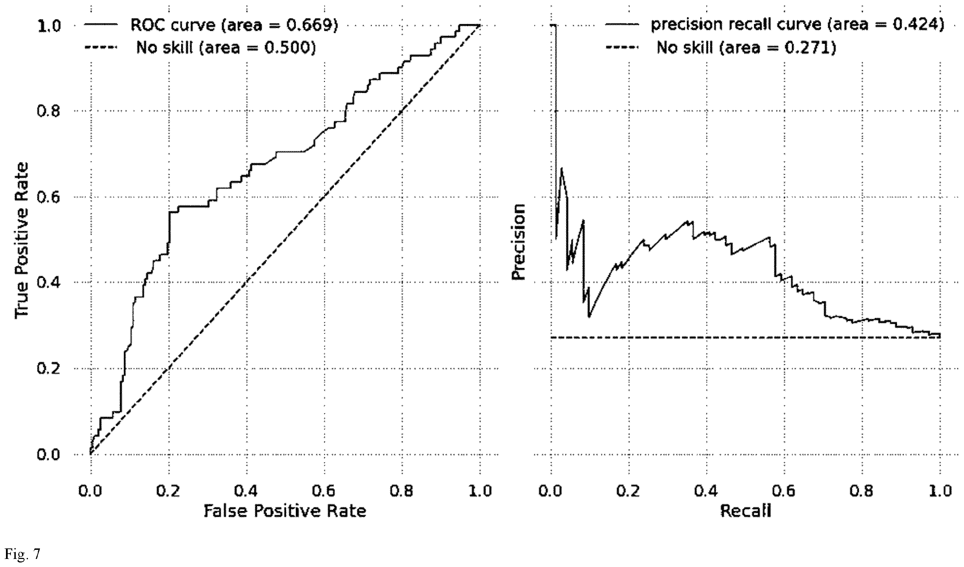

Fig. 7 shows the performance of the trained machine learning model.

## Claims

1. A computer-implemented method of classifying an individual suffering from chronic cough, the method comprising:
- providing a machine learning model, wherein the machine learning model is configured and trained on training data to assign a patient to one of two classes, a first class representing patients who received medications for chronic cough, and a second class representing patients who received placebos, the training data comprising, for each patient of a multitude of patients, information about cough events of the patient before and after starting treatment with the medication or after receiving the placebos, respectively,
- receiving patient data, wherein the patient data comprise information about cough events of the individual before and after initiating therapy for chronic cough,
- inputting the patient data into the trained machine learning model,
- receiving information about which class the individual has been assigned to from the machine learning model,
- outputting the information.

2. The computer-implemented method of claim 1, wherein the machine learning model is or comprises a random forest classifier, an artificial neural network classifier, a support vector machine, a max entropy classifier, an extreme gradient boosting classifier, or a random fern classifier.

3. The computer-implemented method of claim 1 or 2, wherein the machine learning model is or comprises an artificial neural network.

4. The computer-implemented method of any one of claims 1 to 3, wherein each patient and/or the individual suffers from refractory chronic cough.

5. The computer-implemented method of any one of claims 1 to 4, wherein the information about cough events of each patient comprises information about frequencies of cough in a first time period before and in one or more second time periods after starting treatment of the patient with the medication or placebos, respectively.

6. The computer-implemented method of claim 5, wherein the first time period and/or each second time period is in the range of 8 hours to 48 hours.

7. The computer-implemented method of claim 5 or 6, wherein the one or more second time periods cover a period of 20 days to 120 days.

8. The computer-implemented method of any one of claims 1 to 7, wherein the information about cough events of the individual comprises information about frequencies of cough in a first time period before and in one or more second time periods after starting the therapy for chronic cough, wherein the first time period and/or each second time period is in the range of 8 hours to 48 hours and wherein the one or more second time periods cover a period of 10 days to 120 days.

9. The computer-implemented method of any one of claims 1 to 8, wherein the therapy for chronic cough is considered successful if the individual is assigned to the first class and the therapy for chronic cough is considered unsuccessful if the individual is assigned to the second class.

10. The computer-implemented method of any one of claims 1 to 9, wherein the therapy for chronic cough comprises intake of a medication, wherein the individual is considered a non-responder to the medication if the individual is assigned to the second class.

11. The computer-implemented method of any one of claims 1 to 10, wherein the therapy for chronic cough comprises intake of a dose of a medication, wherein the dose is considered to be too low if the individual is assigned to the second class.

12. A computer system comprising:
a processor; and
a memory storing an application program configured to perform, when executed by the processor, an operation, the operation comprising:
- providing a machine learning model, wherein the machine learning model is configured and trained on training data to assign a patient to one of two classes, a first class representing patients who received medications for chronic cough, and a second class representing patients who received placebos, the training data comprising, for each patient of a multitude of patients, information about cough events of the patient before and after starting treatment with the medication or after receiving the placebos, respectively,
- receiving patient data, wherein the patient data comprise information about cough events of the individual before and after initiating therapy for chronic cough,
- inputting the patient data into the trained machine learning model,
- receiving information about which class the individual has been assigned to from the machine learning model,
- outputting the information.

13. A non-transitory computer readable storage medium having stored thereon software instructions that, when executed by a processor of a computer system, cause the computer system to execute the following steps:
- providing a machine learning model, wherein the machine learning model is configured and trained on training data to assign a patient to one of two classes, a first class representing patients who received medications for chronic cough, and a second class representing patients who received placebos, the training data comprising, for each patient of a multitude of patients, information about cough events of the patient before and after starting treatment with the medication or after receiving the placebos, respectively,
- receiving patient data, wherein the patient data comprise information about cough events of the individual before and after initiating therapy for chronic cough,
- inputting the patient data into the trained machine learning model,
- receiving information about which class the individual has been assigned to from the machine learning model,
- outputting the information.

14. A medication for the treatment of chronic cough, wherein the treatment comprises:
- providing a machine learning model, wherein the machine learning model is configured and trained on training data to assign a patient to one of two classes, a first class representing patients who received medications for chronic cough, and a second class representing patients who received placebos, the training data comprising, for each patient of a multitude of patients, information about cough events of the patient before and after starting treatment with the medication or after receiving the placebos, respectively,
- receiving patient data, wherein the patient data comprise information about cough events of the individual before and after initiating therapy for chronic cough,
- inputting the patient data into the trained machine learning model,
- receiving information about which class the individual has been assigned to from the machine learning model,
- outputting the information.

15. A kit comprising a medication for the treatment of chronic cough and the software instructions of claim 13.
